# DEMANDE DE BREVET EUROPEEN

(11) **EP 4 613 842 A1**
(43) Date de publication de la demande: **10.09.2025**
(21) Numéro de dépôt: 25156180.9
(22) Date de dépôt: 06.02.2025
(51) Int. Cl.: C12M 3/06, C12M 1/00

(54) **PROCÉDÉ D'ANALYSE D'UN OBJET BIOLOGIQUE, MIS EN OEUVRE AU SEIN D'UN SYSTÈME DE PERFUSION MICROFLUIDIQUE**

(30) Priorité: 04.03.2024 FR 2402132
(71) Demandeur: Commissariat à l'Energie Atomique et aux Energies Alternatives, 75015 Paris (FR)
(72) Inventeur: HUT, Marie, 38054 GRENOBLE CEDEX 09 (FR); FOUILLET, Yves, 38054 GRENOBLE CEDEX 09 (FR); AGACHE, Vincent, 38054 GRENOBLE CEDEX 09 (FR)
(74) Mandataire: INNOV-GROUP

(57) **Abrégé**

L'invention concerne un procédé d'analyse d'un objet biologique (O), mis en oeuvre au sein d'un système de perfusion microfluidique, ledit système comprenant un composant microfluidique (1) intégrant un circuit microfluidique (10), ledit circuit microfluidique ayant :
- Un canal principal doté d'un piège hydrodynamique (P_H) recevant ledit objet biologique (O) à perfuser,
- Un canal d'entrée microfluidique (100) connecté sur le canal principal en amont du piège hydrodynamique (P_H),
- Un canal de sortie microfluidique (101) connecté sur le canal principal en aval du piège hydrodynamique (P_H),
- Une première vanne (V1) positionnée sur le canal d'entrée microfluidique (100),
- Un système de pompe formé par une série composée d'au moins une deuxième vanne (V2) et d'une troisième vanne (V3) positionnées sur le canal de sortie microfluidique (101).

## Description

### Domaine technique de l'invention

La présente invention se rapporte à un procédé de perfusion d'un objet biologique, mis en oeuvre au sein d'un système de perfusion microfluidique.

### Etat de la technique

Le principe de venir perfuser un objet biologique en le plaçant dans un piège hydrodynamique a notamment déjà été décrit dans la demande de brevet EP3878942A1**.** Le piège est intégré dans un circuit microfluidique réalisé sur un composant microfluidique, appelé également puce microfluidique ou carte microfluidique.

Des solutions connues utilisent des systèmes externes, tels que des pousses-seringues ou des pompes péristaltiques, pour assurer la circulation du milieu de culture dans le circuit microfluidique et permettre la perfusion de l'objet biologique logé dans le piège hydrodynamique.

Néanmoins, avec de tels systèmes externes, la collecte des sécrétions est effectuée hors du composant microfluidique, ce qui accroit le risque de contaminations biologiques, augmente le risque de perdre une partie de l'échantillon, et surtout empêche une analyse des sécrétions collectées sur la puce, de façon intégrée au système de culture.

Il est cependant possible de venir intégrer un système de pompe péristaltique dans le circuit microfluidique du composant. Cependant, l'actionnement classique de la pompe péristaltique cause souvent un reflux au niveau de la zone de piégeage et soumet ainsi l'organoïde piégé à un cisaillement.

Le but de l'invention est de proposer un procédé de perfusion d'un objet biologique qui limite le risque de contaminations biologiques, et qui permette in fine de collecter les sécrétions générées par l'objet biologique logé dans un piège hydrodynamique, en évitant son cisaillement lors du pompage du milieu de culture vers l'intérieur du circuit microfluidique.

### Exposé de l'invention

Ce but est atteint par un procédé de perfusion d'un objet biologique, mis en oeuvre au sein d'un système de perfusion microfluidique, ledit système comprenant un composant microfluidique intégrant un circuit microfluidique, ledit circuit microfluidique ayant :
- Un canal principal doté d'un piège hydrodynamique recevant ledit objet biologique à perfuser,
- Un canal d'entrée microfluidique connecté sur le canal principal en amont du piège hydrodynamique,
- Un canal de sortie microfluidique connecté sur le canal principal en aval du piège hydrodynamique,
- Une première vanne positionnée sur le canal d'entrée microfluidique,
- Un système de pompe formé par une série composée d'au moins une deuxième vanne et d'une troisième vanne, ce système de pompe étant positionné sur le canal de sortie microfluidique,
- Chaque vanne comportant une cavité et une membrane déformable à l'intérieur de la cavité, ladite membrane étant contrôlable par actionnement pneumatique entre deux états extrêmes, un état ouvert dans lequel elle laisse passer un fluide et un état fermé dans lequel elle bloque le passage du fluide, et au moins dans un état intermédiaire, dit deuxième état intermédiaire, situé entre l'état ouvert et l'état fermé,
- Ledit procédé consistant à transférer ledit fluide du canal d'entrée microfluidique vers le canal de sortie microfluidique en passant à travers le piège hydrodynamique dans lequel est logé ledit objet biologique pour le perfuser, en réalisant une séquence de commande de la première vanne, de la deuxième vanne et de la troisième vanne,
- Chaque transfert du fluide de la deuxième vanne vers la troisième vanne étant réalisé en contrôlant simultanément le déplacement de la membrane de la deuxième vanne et celui de la membrane de la troisième vanne, la deuxième vanne étant contrôlée par un premier gradient de pression dans le sens de la fermeture et la troisième vanne par un deuxième gradient de pression dans le sens de l'ouverture.

De manière avantageuse, le premier gradient de pression et le deuxième gradient de pression sont appliqués de manière symétrique, le premier gradient de pression étant montant et le deuxième gradient de pression étant descendant.

De manière avantageuse, le premier gradient de pression et le deuxième gradient de pression sont appliqués chacun suivant un profil rectiligne ou suivant un profil à plusieurs paliers successifs.

Selon une réalisation particulière, la première vanne, la deuxième vanne et la troisième vanne étant initialement à l'état fermé, ladite séquence de commande comporte :
- Une première étape de commande de la première vanne à l'état ouvert, de la deuxième vanne et de la troisième vanne dans un autre état intermédiaire, dit premier état intermédiaire situé entre l'état ouvert et le deuxième état intermédiaire, pour pomper le fluide à travers le piège hydrodynamique,
- Une deuxième étape de commande de la première vanne à l'état fermé pour pousser le fluide vers le canal de sortie microfluidique, à travers le piège hydrodynamique,
- Une troisième étape de commande de la deuxième vanne du premier état intermédiaire vers le deuxième état intermédiaire et de la troisième vanne du premier état intermédiaire vers l'état ouvert pour créer ledit transfert de fluide de la deuxième vanne vers la troisième vanne, cette commande étant réalisée en appliquant sur la deuxième vanne et la troisième vanne respectivement le premier gradient de pression et le deuxième gradient de pression, de manière à obtenir pendant un même intervalle de temps une fermeture progressive de la deuxième vanne et une ouverture progressive de la troisième vanne,
- Une cinquième étape de commande de la deuxième vanne du deuxième état intermédiaire vers l'état fermé pour pousser le fluide vers la troisième vanne qui est à l'état ouvert.

Selon une autre réalisation particulière, la première vanne, la deuxième vanne et la troisième vanne étant initialement à l'état fermé, ladite séquence de commande comporte :
- Une première étape de commande de la première vanne et de la deuxième vanne à l'état ouvert pour pomper le fluide à travers le piège hydrodynamique,
- Une deuxième étape de commande de la première vanne à l'état fermé pour pousser le fluide vers le canal de sortie microfluidique, à travers le piège hydrodynamique,
- Une troisième étape de commande de la troisième vanne dans le deuxième état intermédiaire,
- Une quatrième étape de commande de la deuxième vanne de l'état ouvert vers le deuxième état intermédiaire et de la troisième vanne du deuxième état intermédiaire vers l'état ouvert pour créer ledit transfert de fluide de la deuxième vanne vers la troisième vanne, cette commande étant réalisée en appliquant sur la deuxième vanne et la troisième vanne respectivement le premier gradient de pression et le deuxième gradient de pression, de manière à obtenir pendant un même intervalle de temps une fermeture progressive de la deuxième vanne et une ouverture progressive de la troisième vanne,
- Une cinquième étape de commande de la deuxième vanne du deuxième état intermédiaire vers l'état fermé pour pousser le fluide vers la troisième vanne qui est à l'état ouvert.

De manière avantageuse, le système de pompe comporte une quatrième vanne située en aval de la troisième vanne, et la quatrième vanne étant maintenue à l'état fermé pendant la première étape de commande, la deuxième étape de commande, la troisième étape de commande, la quatrième étape de commande et la cinquième étape de commande, la séquence de commande comportant :
- Une sixième étape de commande de la quatrième vanne de l'état fermé à l'état ouvert pour autoriser un transfert du fluide de la troisième vanne vers la quatrième vanne, et de commande de la troisième vanne de l'état ouvert vers l'état fermé pour pousser le fluide de la troisième vanne vers la quatrième vanne.

Selon une particularité, la séquence de commande comporte une septième étape de commande de la quatrième vanne, de l'état ouvert à l'état fermé, pour pousser le fluide vers le canal de sortie microfluidique.

L'invention concerne un système de perfusion microfluidique d'un objet biologique, employé pour mettre en oeuvre le procédé de perfusion tel que défini ci-dessus, ledit système comprenant un composant microfluidique intégrant un circuit microfluidique, ledit circuit microfluidique ayant
- Un canal principal doté d'un piège hydrodynamique recevant ledit objet biologique à perfuser,
- Un canal d'entrée microfluidique connecté sur le canal principal en amont du piège hydrodynamique,
- Un canal de sortie microfluidique connecté sur le canal principal en aval du piège hydrodynamique,
- Une première vanne positionnée sur le canal d'entrée microfluidique,
- Un système de pompe formé par une série composée d'au moins une deuxième vanne et d'une troisième vanne, le système de pompe étant positionné sur le canal de sortie microfluidique,
- Chaque vanne comportant une cavité et une membrane déformable à l'intérieur de la cavité, ladite membrane étant contrôlable par actionnement pneumatique entre deux états extrêmes, un état ouvert dans lequel elle laisse passer un fluide et un état fermé dans lequel elle bloque le passage du fluide, et au moins dans un état intermédiaire, dit deuxième état intermédiaire, situé entre l'état ouvert et l'état fermé,
- Ledit système étant configuré pour exécuter la séquence de commande de la première vanne, de la deuxième vanne et de la troisième vanne adaptée pour réaliser le transfert du fluide du canal d'entrée microfluidique vers le canal de sortie microfluidique en passant à travers le piège hydrodynamique dans lequel est logé ledit objet biologique pour le perfuser,
- Dans ladite séquence de commande, chaque transfert du fluide de la deuxième vanne vers la troisième vanne étant réalisé en contrôlant simultanément le déplacement de la membrane de la deuxième vanne et celui de la membrane de la troisième vanne, la deuxième vanne étant contrôlée par un premier gradient de pression dans le sens de la fermeture et la troisième vanne par un deuxième gradient de pression dans le sens de l'ouverture.

De manière avantageuse, le premier gradient de pression et le deuxième gradient de pression sont appliqués de manière symétrique, le premier gradient de pression étant montant et le deuxième gradient de pression étant descendant.

De manière avantageuse, le premier gradient de pression et le deuxième gradient de pression sont appliqués chacun suivant un profil rectiligne ou suivant un profil à plusieurs paliers successifs.

Selon une réalisation particulière, la première vanne, la deuxième vanne et la troisième vanne étant initialement à l'état fermé, ladite séquence de commande comporte :
- Une première étape de commande de la première vanne à l'état ouvert, de la deuxième vanne et de la troisième vanne dans un autre état intermédiaire, dit premier état intermédiaire situé entre l'état ouvert et le deuxième état intermédiaire, pour pomper le fluide à travers le piège hydrodynamique,
- Une deuxième étape de commande de la première vanne à l'état fermé pour pousser le fluide vers le canal de sortie microfluidique, à travers le piège hydrodynamique,
- Une troisième étape de commande de la deuxième vanne du premier état intermédiaire vers le deuxième état intermédiaire et de la troisième vanne du premier état intermédiaire vers l'état ouvert pour créer ledit transfert de fluide de la deuxième vanne vers la troisième vanne, cette commande étant réalisée en appliquant sur la deuxième vanne et la troisième vanne respectivement le premier gradient de pression et le deuxième gradient de pression, de manière à obtenir pendant un même intervalle de temps une fermeture progressive de la deuxième vanne et une ouverture progressive de la troisième vanne,
- Une cinquième étape de commande de la deuxième vanne du deuxième état intermédiaire vers l'état fermé pour pousser le fluide vers la troisième vanne qui est à l'état ouvert.

Selon une autre réalisation particulière, la première vanne, la deuxième vanne et la troisième vanne étant initialement à l'état fermé, ladite séquence de commande comporte :
- Une première étape de commande de la première vanne et de la deuxième vanne à l'état ouvert pour pomper le fluide à travers le piège hydrodynamique,
- Une deuxième étape de commande de la première vanne à l'état fermé pour pousser le fluide vers le canal de sortie microfluidique, à travers le piège hydrodynamique,
- Une troisième étape de commande de la troisième vanne dans le deuxième état intermédiaire,
- Une quatrième étape de commande de la deuxième vanne de l'état ouvert vers le deuxième état intermédiaire et de la troisième vanne du deuxième état intermédiaire vers l'état ouvert pour créer ledit transfert de fluide de la deuxième vanne vers la troisième vanne, cette commande étant réalisée en appliquant sur la deuxième vanne et la troisième vanne respectivement le premier gradient de pression et le deuxième gradient de pression, de manière à obtenir pendant un même intervalle de temps une fermeture progressive de la deuxième vanne et une ouverture progressive de la troisième vanne,
- Une cinquième étape de commande de la deuxième vanne du deuxième état intermédiaire vers l'état fermé pour pousser le fluide vers la troisième vanne qui est à l'état ouvert.

De manière avantageuse, le système de pompe comporte une quatrième vanne située en aval de la troisième vanne, la quatrième vanne étant maintenue à l'état fermé pendant la première étape de commande, la deuxième étape de commande, la troisième étape de commande, la quatrième étape de commande et la cinquième étape de commande, la séquence de commande comportant :
- Une sixième étape de commande de la quatrième vanne de l'état fermé à l'état ouvert pour autoriser un transfert du fluide de la troisième vanne vers la quatrième vanne, et de commande de la troisième vanne de l'état ouvert vers l'état fermé pour pousser le fluide de la troisième vanne vers la quatrième vanne.

Selon une particularité, la séquence de commande comporte une septième étape de commande de la quatrième vanne, de l'état ouvert à l'état fermé, pour pousser le fluide vers le canal de sortie microfluidique.

### Brève description des figures

D'autres caractéristiques et avantages vont apparaître dans la description détaillée qui suit faite en regard des dessins annexés dans lesquels :
- La figure 1 représente de manière schématique le système de perfusion microfluidique de l'invention ;
- La figure 2 représente le système de piège hydrodynamique employé au sein du système de perfusion microfluidique de l'invention ;
- Les figures 3A à 3C montrent un exemple de réalisation d'une vanne microfluidique employée dans le circuit microfluidique et illustrent son principe de fonctionnement ;
- Les figures 4A et 4B illustrent les différentes étapes de mise en oeuvre du procédé de perfusion de l'invention, selon une première variante de réalisation ;
- La figure 5 représente un chronogramme illustrant les étapes de mise en oeuvre du procédé de perfusion de l'invention, selon la première variante de réalisation ;
- Les figures 6A et 6B illustrent les différentes étapes de mise en oeuvre du procédé de perfusion de l'invention, selon une deuxième variante de réalisation ;
- La figure 7 représente un chronogramme illustrant les étapes de mise en oeuvre du procédé de perfusion de l'invention, selon la deuxième variante de réalisation ;

### Description détaillée d'au moins un mode de réalisation

L'invention s'applique à la perfusion d'un objet biologique O. L'objet biologique O est par exemple un agrégat de cellules. Par agrégat de cellules, on entend, selon l'invention, l'auto-assemblage d'un ou plusieurs types de cellules en trois dimensions. Un tel agrégat de cellules peut notamment s'appeler sphéroïde, organoïde, neuro-sphère. Cet agrégat peut également être un îlot de Langerhans. Dans la suite de la description, on utilisera de manière générique le terme « objet biologique » pour évoquer un tel agrégat, ce terme étant classiquement employé dans le domaine de la culture de cellules vivantes. De manière non limitative, un tel objet biologique O peut par exemple présenter un diamètre allant de quelques dizaines de µm à quelques centaines de µm.

Dans la suite de la description, les termes « amont » et « aval » sont à comprendre en tenant compte du sens du flux fluidique dans le circuit microfluidique.

Il faut comprendre que l'invention ne se limite pas la perfusion d'un objet biologique mais il faut noter qu'elle présente certains avantages pour cette application.

### Composant microfluidique

### Figure 1

Le procédé est mis en oeuvre dans un système de perfusion microfluidique qui comporte un composant microfluidique 1, également appelé puce microfluidique, carte microfluidique ou cartouche microfluidique.

Un tel composant microfluidique 1 est réalisé sous la forme d'un élément monobloc intégrant un circuit microfluidique 10 qui comporte les éléments adaptés pour mettre en oeuvre toutes les opérations de circulation du fluide dans le circuit microfluidique 10.

Le composant microfluidique 1 est souvent réalisé par l'assemblage de plusieurs couches entre elles. Ses couches sont par exemple assemblées entre elles par scellement thermique. Chaque couche peut être usinée de manière à y créer une partie du réseau microfluidique du composant.

Le composant microfluidique 1 présente par exemple une épaisseur comprise entre 2mm et 12mm.

Dans le système de perfusion microfluidique, le composant microfluidique 1 est associé à un système d'actionnement pneumatique 4, capable d'appliquer une pression d'actionnement (une pression positive P+, une pression négative P-, une pression nulle P_int_1 ou une pression intermédiaire P_int_2) sur chaque capsule microfluidique (voir ci-après) apte à être commandée. Le système d'actionnement pneumatique 4 est commandé par une unité de traitement et de commande UC, configurée pour exécuter une séquence de commande comportant les étapes du procédé d'analyse de l'invention. Ainsi, lorsqu'une capsule microfluidique est commandée dans un état ouvert, fermé ou intermédiaire, le système d'actionnement pneumatique 4 applique la pression voulue sur commande de l'unité de traitement et de commande UC. Dans le cadre de l'invention, on verra ci-après que le système d'actionnement pneumatique 4 est capable d'appliquer une pression intermédiaire P_int_2 adaptée pour conférer à la capsule microfluidique un état intermédiaire, situé entre son état ouvert et son état fermé.

### Circuit microfluidique

### Figure 1

### Figure 2

Le circuit microfluidique 10 du composant comporte au moins un premier canal d'entrée microfluidique 100 et au moins un premier canal de sortie microfluidique 101.

Le circuit microfluidique comporte un premier réservoir R1 destiné à recevoir le milieu de culture utilisé pour la perfusion de l'objet biologique O. Ce premier réservoir R1 est connecté sur le premier canal d'entrée microfluidique 100.

Le circuit microfluidique comporte une première vanne microfluidique V1 agencée sur le premier canal d'entrée microfluidique 100 pour contrôler la sortie du milieu de culture en provenance du premier réservoir R1.

Le circuit microfluidique 10 comporte également un système de pompe, de type péristaltique, agencé sur le premier canal de sortie microfluidique 101 et commandé pour contrôler l'injection de fluide dans le circuit microfluidique 10.

Entre le premier canal d'entrée microfluidique 100 et le premier canal de sortie microfluidique 101, le circuit microfluidique 10 comporte un canal principal doté d'un système de piège hydrodynamique P_H.

Le principe de piégeage hydrodynamique a notamment été décrit dans la publication suivante :
- Tan, W. H., & Takeuchi, S. (2007). A trap-and-release integrated microfluidic system for dynamic microarray applications. Proceedings of the National Academy of Sciences, 104(4), 1146-1151*.*

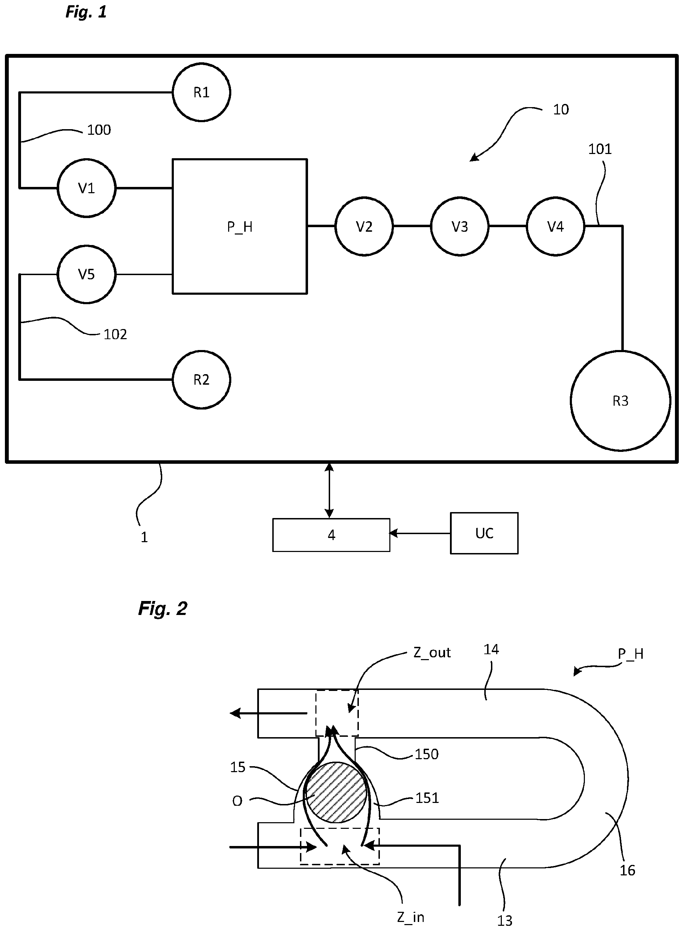

En référence à la figure 2, le système de piège hydrodynamique P_H comporte une zone d'entrée microfluidique Z_in et une zone de sortie microfluidique Z_out.

Entre sa zone d'entrée microfluidique Z_in et sa zone de sortie microfluidique Z_out, le système comporte deux canaux latéraux 13, 14 reliés entre eux à la fois par un canal central 15 et par un canal secondaire 16 en formant un coude.

Le canal central 15 comporte un étranglement 150 ou restriction et une cavité 151 de piégeage hydrodynamique d'un objet biologique réalisée en amont de cet étranglement 150.

Le canal secondaire 16 forme une dérivation par rapport au canal central 15.

Selon l'invention, le canal central 15 est configuré pour que sa section résultante après occultation par l'objet biologique O induise une perte de charge plus importante à travers le canal central 15 que la perte de charge présente à travers le canal secondaire 16. Plus précisément, les résistances hydrauliques du canal central 15 et du canal secondaire 16, notées respectivement Rₚ et Rₛ sont, avant piégeage de l'objet biologique, telles que Rₚ < Rₛ et, après piégeage de l'objet biologique, telles que Rₚ > Rₛ. Ces inégalités garantissent le principe de fonctionnement hydrodynamique du piège. Bien entendu, plusieurs configurations et plusieurs schémas sont envisageables pour remplir ces conditions de fonctionnement.

La zone d'entrée microfluidique Z_in du système de piège hydrodynamique P_H est définie comme étant située en amont de la cavité destinée à recevoir l'objet biologique, en considérant le sens du flux fluidique, et la zone de sortie microfluidique Z_out du système de piège hydrodynamique P_H est définie comme étant située en aval de la cavité 151 destinée à recevoir l'objet biologique O, en considérant le sens du flux fluidique.

Le premier canal d'entrée microfluidique 100 du circuit microfluidique 10 vient se connecter sur la zone d'entrée microfluidique Z_in du système de piège hydrodynamique et le premier canal de sortie microfluidique 101 du circuit microfluidique 10 vient se connecter sur la zone de sortie microfluidique Z_out du système de piège hydrodynamique P_H.

Le circuit microfluidique 10 comporte avantageusement un deuxième réservoir R2 destiné à recevoir l'objet biologique O à analyser.

Le circuit microfluidique 10 comporte avantageusement un deuxième canal microfluidique d'entrée 102. Le deuxième réservoir R2 est connecté sur ce deuxième canal microfluidique d'entrée 102. Le deuxième canal fluidique d'entrée 102 est placé en parallèle du premier canal microfluidique d'entrée 100 et vient se connecter sur la zone d'entrée microfluidique Z_in du système de piège hydrodynamique pour relier le deuxième réservoir R2 au système de piège hydrodynamique P_H. Une vanne V5 est également positionnée sur le deuxième canal microfluidique d'entrée 102 afin de contrôler l'injection de l'objet biologique O dans le système de piège hydrodynamique P_H.

Le circuit microfluidique 10 est ainsi configuré pour que le système de piège hydrodynamique P_H puisse recevoir l'objet biologique O ou le milieu de culture, en contrôlant les vannes V1, V5 respectives et le système de pompe situé en aval.

Le système de pompe comporte au moins trois vannes microfluidiques V2, V3, V4 connectées en série sur le canal fluidique de sortie. Par connectés en « série », on entend que chaque vanne est reliée directement à une autre vanne dans la série par un unique canal microfluidique, de sorte que le flux fluidique peut passer directement d'une vanne à l'autre dans la série.

Le circuit microfluidique 10 comporte avantageusement un troisième réservoir R3 agencé en aval du système de pompe, sur le canal microfluidique de sortie 101, ce troisième réservoir R3 étant destiné à recevoir les sécrétions générées par l'objet biologique O lorsqu'il est perfusé par le milieu de culture.

### Vanne microfluidique

### Figure 3A

### Figure 3B

### Figure 3C

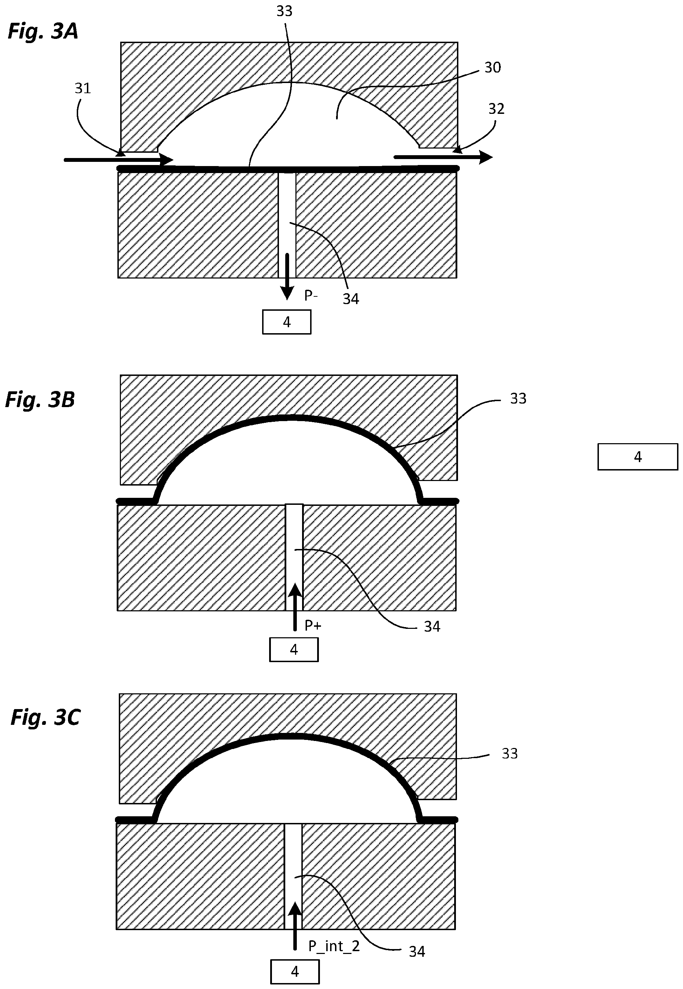

Dans le cadre de l'invention, chaque vanne (V1 à V5) est par exemple conçue selon l'architecture représentée sur les figures 3A à 3C.

La vanne se présente par exemple sous la forme d'une capsule microfluidique.

La capsule microfluidique comporte une chambre 30 ou cavité fluidique dans laquelle débouche un canal d'entrée 31 et dont ressort un canal de sortie 32. Une membrane 33 déformable est commandée entre deux positions pour conférer deux états distincts à la vanne, un premier état ouvert (S0) dans lequel le canal d'entrée 31 communique avec le canal de sortie 32 via la chambre 30, autorisant un transfert de fluide (figure 3A), et un deuxième état fermé (S3) dans lequel la membrane 33 bloque la communication entre les deux canaux, empêchant l'écoulement de fluide et le remplissage de la chambre (figure 3B). La commande de la membrane 33 entre ses deux états est réalisée à l'aide du système d'actionnement pneumatique 4, par exemple en exerçant sur elle une pression positive P+ (pour fermer la vanne, figure 3B) ou une pression négative P- (pour ouvrir la vanne, figure 3A) via un canal d'actionnement 34 spécifique intégré dans le composant microfluidique. Par ailleurs, lorsque le système d'actionnement applique une pression intermédiaire ou même qu'aucune pression n'est appliquée (pression nulle), la vanne est dans un état intermédiaire, situé entre son état ouvert et son état fermé. Pour la suite de la description, on définit deux états intermédiaires, désignés S1 et S2 ci-après. Ces états intermédiaires permettent de garantir des passages moins brusques de l'état ouvert à l'état fermé ou inversement. Sur la figure 3C, on vient appliquer une pression intermédiaire P_int_2 permettant de fermer la vanne, cette pression étant inférieure à celle pour obtenir la fermeture de la vanne (P+).

A titre d'exemple, la pression positive P+ appliquée pour fermer la vanne (état S3) est de 450mbars, la pression négative P- appliquée pour ouvrir la vanne (état S0) est de - 150mbars, la pression intermédiaire P_int_1 est par exemple nulle et la pression intermédiaire P_int_2 est par exemple fixée à 150mbars. Bien entendu, ces valeurs pourront varier selon l'architecture du système.

Comme indiqué ci-dessus, le composant microfluidique 1 peut être réalisé par un assemblage multicouches dans lequel la membrane déformable 33 forme une couche intermédiaire prise entre deux couches du composant. La membrane 33 peut être collée ou fixée par de l'adhésif double-face prédécoupé ou par un traitement plasma. Actuellement la membrane 33 déformable est souvent réalisée dans un matériau hyperélastique à base de silicone, tel que le polydiméthylsiloxane (PDMS), ou d'élastomère comme l'Ecoflex (marque déposée).

Dans le cadre de l'architecture décrite ci-dessus, le principe de l'invention consiste à mettre en oeuvre une séquence de commande des différentes vannes (V1 à V4) pour permettre la perfusion de l'objet biologique O logé dans le système de piège hydrodynamique P_H à l'aide du milieu de culture en limitant les risques de cisaillement de l'objet biologique O. Autrement dit, la séquence est adaptée pour pomper le milieu de culture de l'amont vers l'aval, à travers le piège hydrodynamique P_H dans lequel est logé l'objet biologique O, et récupérer les sécrétions générées par l'objet biologique O sous perfusion. D'autres applications seraient bien entendu envisageables.

La séquence est appliquée aux vannes V1, V2, V3, et V4 du circuit microfluidique. D'autres applications seraient bien entendu envisageables.

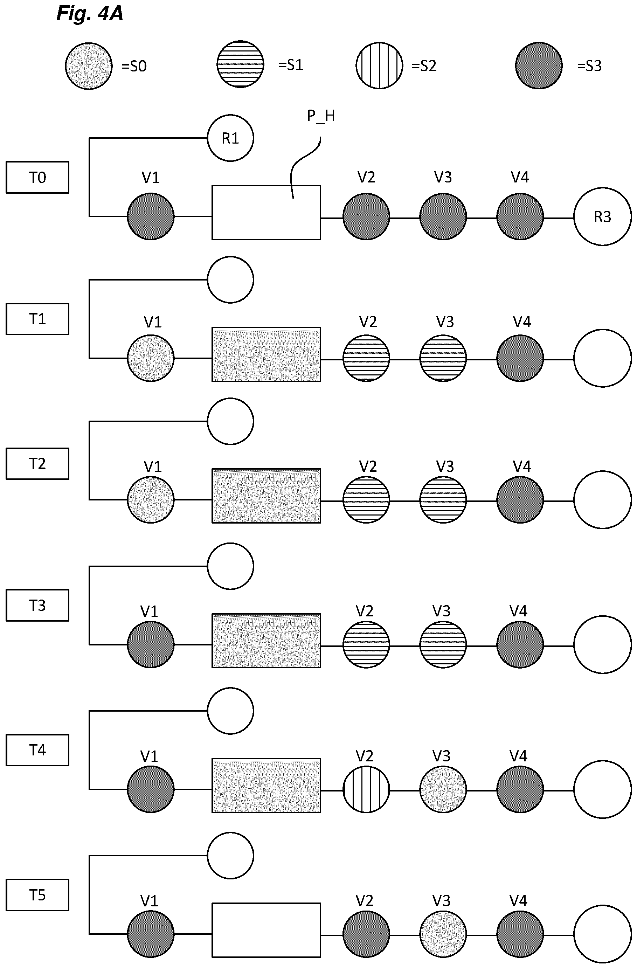

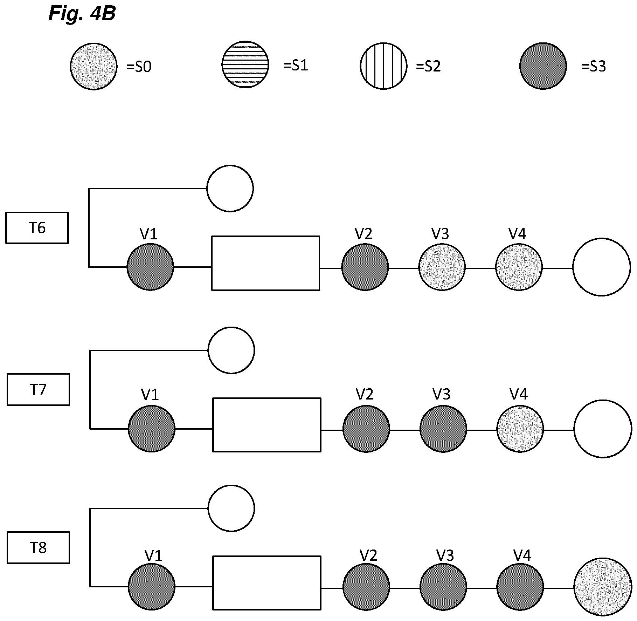

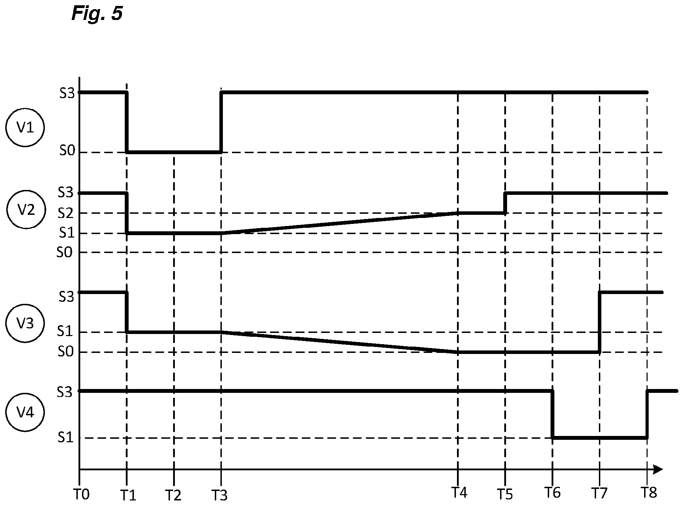

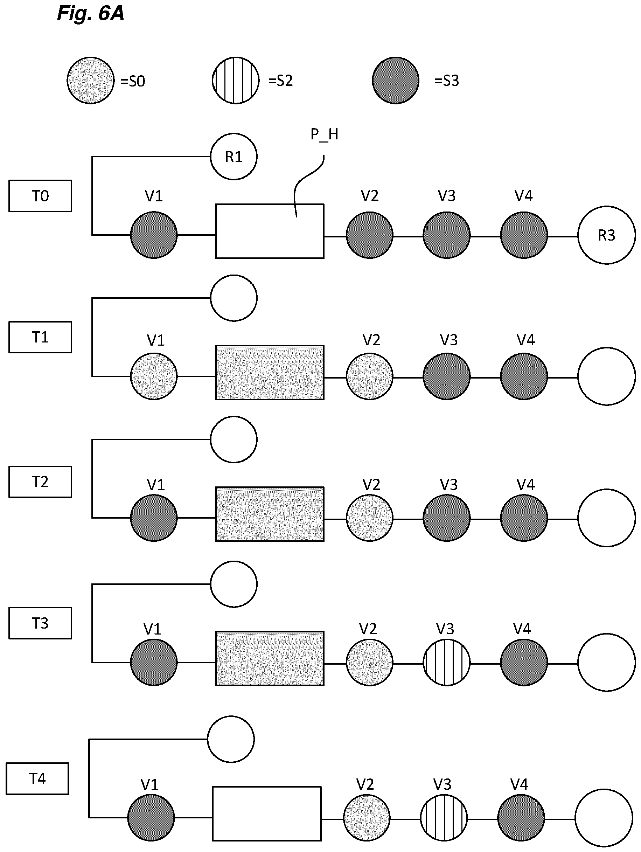

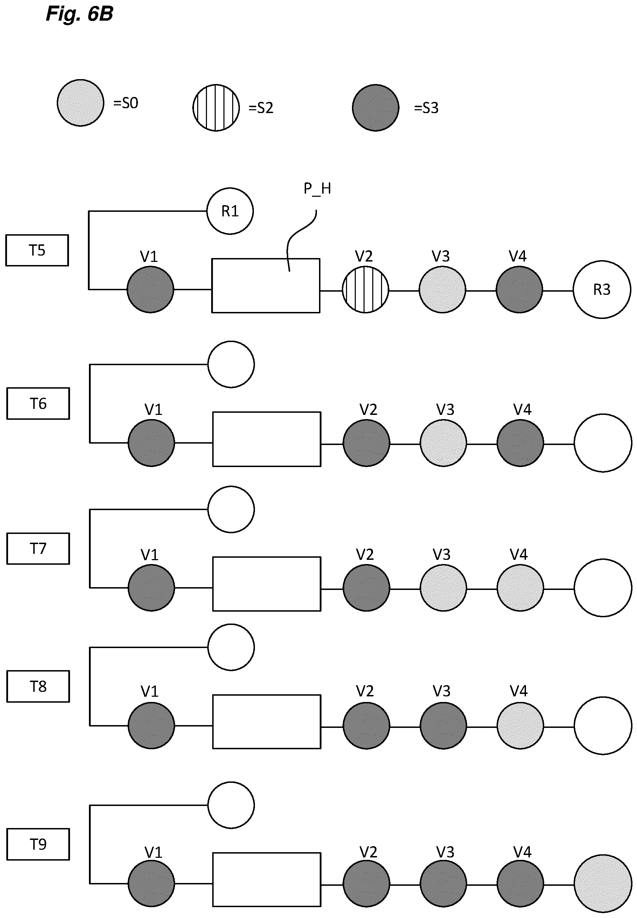

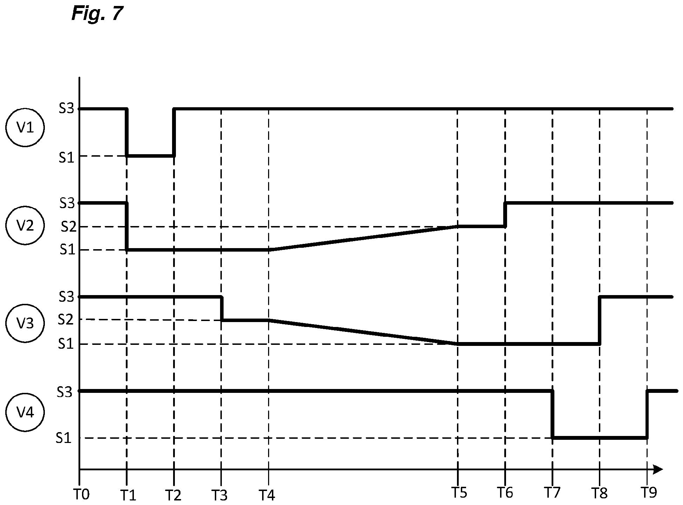

Les figures 4A, 4B et 5 montrent une première variante de réalisation de la séquence de commande et les figures 6A, 6B et 7 montrent une deuxième variante de réalisation de la séquence de commande.

Sur ces figures, l'état ouvert est désigné S0, le premier état intermédiaire est désigné S1, le deuxième état intermédiaire est désigné S2, et l'état fermé est désigné S3. Dans chaque séquence, les différents instants T0 à T9 sont des instants distincts, qui se suivent dans un ordre chronologique. Il faut noter que la durée présente entre deux instants consécutifs n'est pas toujours identique.

Dans les séquences décrites ci-dessous, il faut noter que l'objet biologique O a été préalablement piégé dans le système de piège hydrodynamique P_H.

### Séquence de commande - première variante

### Figure 4A

### Figure 4B

### Figure 5

Pour cette première variante, les différentes étapes sont les suivantes :

A partir de T0 : Les vannes V1, V2, V3, V4 sont maintenues à l'état fermé S3 par le système d'actionnement pneumatique, en appliquant la pression P+ (450mbars).

A T1 : Le système d'actionnement pneumatique commande la vanne V1 à l'état ouvert S1 (P- à -150mbars). La vanne V2 et la vanne V3 sont commandées à l'état intermédiaire S1, permettant leur ouverture partielle et de limiter le flux à travers le système de piège hydrodynamique P_H. Le milieu de culture est ainsi pompé hors du réservoir R1 et vient perfuser l'objet biologique O en passant au travers du système de piège hydrodynamique P_H.

Entre T1 et T3 : La situation reste identique.

A T3 : La vanne V1 est commandée à l'état fermé S3. A cet instant, la vanne V1 est donc à l'état fermé S3, la vanne V2 et la vanne V3 sont dans l'état intermédiaire S1.

Entre T3 et T4 : La vanne V2 est commandée progressivement de l'état intermédiaire S1 à l'état intermédiaire S2 et, dans le même temps (sur la même période), la vanne V3 est commandée progressivement de l'état intermédiaire S1 à l'état ouvert S0. Par le terme « progressivement », on entend que le changement d'état n'est pas brusque et est réalisée par application d'une variation de la pression d'actionnement, en appliquant un gradient de pression suivant une courbe avantageusement linéaire ayant une pente supérieure à 0 et inférieure à 1. La variation de la pression d'actionnement de la vanne peut également être appliquée par paliers successifs de pression.

A T4 : La vanne V2 est à l'état intermédiaire S2 et la vanne V3 est à l'état ouvert S0. De cette manière, le fluide est transféré progressivement de la vanne V2 vers la vanne V3, en évitant tout reflux vers l'amont où se trouve l'objet biologique O.

A T5 : La vanne V2 est commandée vers l'état fermé S3 pour terminer le transfert de fluide vers la vanne V3 qui est à l'état ouvert S0. L'augmentation de la pression au niveau de la vanne V2 permet d'assurer une fermeture étanche par rapport à la partie aval du circuit microfluidique 10. Ce passage de l'état intermédiaire S2 à l'état fermé S3 présente l'avantage de ne pas être trop brutal.

A T6 : La vanne V4 est commandée de l'état fermé S3 à l'état ouvert S0 pour autoriser le transfert de fluide en provenance de la vanne V3 qui est toujours dans l'état ouvert S0.

A T7 : La vanne V3 est commandée à l'état fermé S3 pour transférer le fluide vers la vanne V4. Comme la vanne V2 est maintenue à l'état fermé S3, on évite tout reflux vers l'amont.

A T8 : La vanne V4 est commandée à l'état fermé S3 pour pousser le fluide vers l'aval, par exemple vers le réservoir de collecte R3 (si celui-ci est présent).

Cette séquence peut être reproduite tant que l'objet biologique O est amené à être perfusé par le milieu de culture et que l'on souhaite récupérer les sécrétions générées.

### Séquence de commande - deuxième variante

### Figure 6A

### Figure 6B

### Figure 7

Dans cette deuxième variante de réalisation, on n'utilise que les trois états distincts, S0, S2 et S3 définis ci-dessus.

Pour cette deuxième variante, les différentes étapes sont les suivantes :
A partir de T0 : Les vannes V1, V2, V3, V4 sont maintenues à l'état fermé S3 par le système d'actionnement pneumatique, en appliquant la pression P+ (450mbars).

A T1 : Le système d'actionnement pneumatique commande la vanne V1 et la vanne V2 à l'état ouvert sa (P- à -150mbars). La vanne V3 reste à l'état fermé S3. Le milieu de culture est ainsi pompé hors du réservoir R1 et vient perfuser l'objet biologique O en passant au travers du système piège hydrodynamique P_H.

A T2 : La vanne V1 est repassée à l'état fermé S3 pour prévenir le reflux provoqué par la fermeture de la vanne V2 prévue dans la suite de la séquence.

A T3 : La vanne V3 est commandée dans l'état intermédiaire S2 (P+ à 150mbars). Cette commande pourrait également être réalisée à l'instant T2.

A T4 : La vanne V1 est donc à l'état fermé S3, la vanne V2 est à l'état ouvert S0 et la vanne V3 dans l'état intermédiaire S2.

Entre T4 et T5 : La vanne V2 est commandée progressivement de l'état ouvert sa à l'état intermédiaire S2 et, dans le même temps (sur la même période), la vanne V3 est commandée progressivement de l'état intermédiaire S2 à l'état ouvert S0. Par le terme « progressivement », on entend que le changement d'état n'est pas brusque et est réalisée par application d'une variation de la pression d'actionnement, en appliquant un gradient de pression suivant une courbe avantageusement linéaire ayant une pente supérieure à 0 et inférieure à 1. La variation de la pression d'actionnement de la vanne peut également être appliquée par paliers successifs de pression.

A T5 : La vanne V2 est à l'état intermédiaire S2 et la vanne V3 est à l'état ouvert S0. De cette manière, le fluide est transféré progressivement de la vanne V2 vers la vanne V3, en évitant tout reflux vers l'amont où se trouve l'objet biologique O.

A T6 : La vanne V2 est commandée vers l'état fermé S3 pour terminer le transfert de fluide vers la vanne V3 qui est à l'état ouvert S0. L'augmentation de la pression au niveau de la vanne V2 permet d'assurer une fermeture étanche par rapport à la partie aval du circuit microfluidique 10.

A T7 : La vanne V4 est commandée de l'état fermé S3 à l'état ouvert S0 pour autoriser le transfert de fluide en provenance de la vanne V3 qui est toujours dans l'état ouvert S0.

A T8 : La vanne V3 est commandée à l'état fermé S3 pour transférer le fluide vers la vanne V4. Comme la vanne V2 est maintenue à l'état fermé S3, on évite tout reflux vers l'amont.

A T9 : La vanne V4 est commandée à l'état fermé S3 pour pousser le fluide vers l'aval, par exemple vers le réservoir de collecte R3 (si celui-ci est présent).

Cette séquence peut être reproduite tant que l'objet biologique O est amené à être perfusé par le milieu de culture et que l'on souhaite récupérer les sécrétions générées.

L'invention mise en oeuvre dans les deux séquences décrites ci-dessus réside principalement dans le transfert doux de fluide de la vanne V2 vers la vanne V3, en évitant tout reflux de fluide vers l'amont. L'application d'une variation progressive de la pression d'actionnement sur les vannes V2 et V3 permet d'éviter ce reflux. Par le terme « progressive » on entend qu'elle est réalisée sur une durée au moins dix fois plus longue que pour un changement d'état brusque d'une vanne. Dans ce dernier cas, le changement d'état est effectué par application d'un front de pression montant ou descendant (selon le changement d'état opéré) qui dure moins d'une seconde, ou même moins de 100 millisecondes. L'utilisation du terme de gradient permet d'illustrer ce changement progressif d'état, par opposition à un changement d'état brutal.

Dans la première variante de séquence, la variation de pression appliquée à la vanne V2 pour la commander de l'état intermédiaire S1 vers l'état intermédiaire S2 et la variation de pression appliquée à la vanne V3 pour la commander de l'état intermédiaire S1 à l'état ouvert S0 sont réalisées suivant des profils symétriques (que ce soit suivant un profil linéaire de pente donnée ou suivant un profil en escalier).

Cette séquence permet de générer des débits fluidiques faibles à travers la zone de piégeage et donc de limiter les cisaillements autour de l'objet biologique piégé.

De même, dans la deuxième variante de la séquence, la variation de pression appliquée à la vanne V2 pour la commander de l'état ouvert S0 vers l'état intermédiaire S2 et la variation de pression appliquée à la vanne V3 pour la commander de l'état intermédiaire S2 à l'état ouvert S0 sont réalisées suivant des profils symétriques (que ce soit suivant un profil linéaire de pente donnée ou suivant un profil en escalier).

Dans les deux séquences décrites ci-dessus, on note que la variation de pression appliquée lors de l'application des gradients de pression reste identique (ici 150mbars). Dans la première variante, on utilise deux états intermédiaires (S1 et S2) pour assurer des changements d'état moins brusques et donc limiter les cisaillements sur l'objet biologique O.

L'invention permet ainsi d'éviter tout reflux de fluide en amont lors du transfert vers l'aval et ainsi de limiter les risques de cisaillement et donc d'endommagement de l'objet biologique O.

## Revendications

1. Procédé de perfusion d'un objet biologique (O) , mis en oeuvre au sein d'un système de perfusion microfluidique, ledit système comprenant un composant microfluidique (1) intégrant un circuit microfluidique (10), ledit circuit microfluidique ayant :
- Un canal principal doté d'un piège hydrodynamique (P_H) recevant ledit objet biologique (O) à perfuser,
- Un canal d'entrée microfluidique (100) connecté sur le canal principal en amont du piège hydrodynamique (P_H),
- Un canal de sortie microfluidique (101) connecté sur le canal principal en aval du piège hydrodynamique (P_H),
- Une première vanne (V1) positionnée sur le canal d'entrée microfluidique (100),
- Un système de pompe formé par une série composée d'au moins une deuxième vanne (V2) et d'une troisième vanne (V3) positionnées sur le canal de sortie microfluidique (101),
- Chaque vanne comportant une cavité (30) et une membrane (33) déformable à l'intérieur de la cavité, ladite membrane (33) étant contrôlable par actionnement pneumatique entre deux états extrêmes, un état ouvert (S0) dans lequel elle laisse passer un fluide et un état fermé (S3) dans lequel elle bloque le passage du fluide, et au moins dans un état intermédiaire, dit deuxième état intermédiaire (S2), situé entre l'état ouvert et l'état fermé,
- Ledit procédé consistant à transférer ledit fluide du canal d'entrée microfluidique (100) vers le canal de sortie microfluidique (101) en passant à travers le piège hydrodynamique (P_H) dans lequel est logé ledit objet biologique pour le perfuser, en réalisant une séquence de commande de la première vanne (V1), de la deuxième vanne (V2) et de la troisième vanne (V3),
- **Caractérisé en ce que** chaque transfert du fluide de la deuxième vanne (V2) vers la troisième vanne (V3) est réalisé en contrôlant simultanément le déplacement de la membrane de la deuxième vanne (V2) et celui de la membrane de la troisième vanne (V3), la deuxième vanne (V2) étant contrôlée par un premier gradient de pression dans le sens de la fermeture et la troisième vanne (V3) par un deuxième gradient de pression dans le sens de l'ouverture.

2. Procédé selon la revendication 1, **caractérisé en ce que** le premier gradient de pression et le deuxième gradient de pression sont appliqués de manière symétrique, le premier gradient de pression étant montant et le deuxième gradient de pression étant descendant.

3. Procédé selon la revendication 1 ou 2, **caractérisé en ce que** le premier gradient de pression et le deuxième gradient de pression sont appliqués chacun suivant un profil rectiligne ou suivant un profil à plusieurs paliers successifs.

4. Procédé selon l'une des revendications 1 à 3, **caractérisé en ce que**, la première vanne (V1), la deuxième vanne (V2) et la troisième vanne (V3) étant initialement à l'état fermé (S3), ladite séquence de commande comporte :
- Une première étape de commande de la première vanne à l'état ouvert, de la deuxième vanne (V2) et de la troisième vanne (V3) dans un autre état intermédiaire, dit premier état intermédiaire (S1) situé entre l'état ouvert (S0) et le deuxième état intermédiaire (S2), pour pomper le fluide à travers le piège hydrodynamique (P_H),
- Une deuxième étape de commande de la première vanne (V1) à l'état fermé (S3) pour pousser le fluide vers le canal de sortie microfluidique (101), à travers le piège hydrodynamique (P_H),
- Une troisième étape de commande de la deuxième vanne (V2) du premier état intermédiaire (S1) vers le deuxième état intermédiaire (S2) et de la troisième vanne (V3) du premier état intermédiaire (S1) vers l'état ouvert (S0) pour créer ledit transfert de fluide de la deuxième vanne (V2) vers la troisième vanne (V3), cette commande étant réalisée en appliquant sur la deuxième vanne (V2) et la troisième vanne (V3) respectivement le premier gradient de pression et le deuxième gradient de pression, de manière à obtenir pendant un même intervalle de temps une fermeture progressive de la deuxième vanne (V2) et une ouverture progressive de la troisième vanne (V3),
- Une cinquième étape de commande de la deuxième vanne (V2) du deuxième état intermédiaire (S2) vers l'état fermé (S3) pour pousser le fluide vers la troisième vanne (V3) qui est à l'état ouvert (S0).

5. Procédé selon l'une des revendications 1 à 3, **caractérisé en ce que**, la première vanne (V1), la deuxième vanne (V2) et la troisième vanne (V3) étant initialement à l'état fermé, ladite séquence de commande comporte :
- Une première étape de commande de la première vanne et de la deuxième vanne à l'état ouvert pour pomper le milieu de culture à travers le piège hydrodynamique (P_H),
- Une deuxième étape de commande de la première vanne (V1) à l'état fermé (S3) pour pousser le milieu de culture vers le canal de sortie microfluidique (101), à travers le piège hydrodynamique (P_H),
- Une troisième étape de commande de la troisième vanne (V3) dans le deuxième état intermédiaire (S2),
- Une quatrième étape de commande de la deuxième vanne (V2) de l'état ouvert (S0) vers le deuxième état intermédiaire (S2) et de la troisième vanne (V3) du deuxième état intermédiaire (S2) vers l'état ouvert (S0) pour créer ledit transfert de fluide de la deuxième vanne (V2) vers la troisième vanne (V3), cette commande étant réalisée en appliquant sur la deuxième vanne et la troisième vanne respectivement le premier gradient de pression et le deuxième gradient de pression, de manière à obtenir pendant un même intervalle de temps une fermeture progressive de la deuxième vanne (V2) et une ouverture progressive de la troisième vanne (V3),
- Une cinquième étape de commande de la deuxième vanne (V2) du deuxième état intermédiaire (S2) vers l'état fermé (S3) pour pousser le fluide vers la troisième vanne (V3) qui est à l'état ouvert.

6. Procédé selon la revendication 4 ou 5, **caractérisé en ce que** le système de pompe comporte une quatrième vanne (V4) située en aval de la troisième vanne (V3) et **en ce que** la quatrième vanne (V4) est maintenue à l'état fermé (S3) pendant la première étape de commande, la deuxième étape de commande, la troisième étape de commande, la quatrième étape de commande et la cinquième étape de commande et **en ce que** la séquence de commande comporte :
- Une sixième étape de commande de la quatrième vanne (V4) de l'état fermé (S3) à l'état ouvert (S0) pour autoriser un transfert du fluide de la troisième vanne (V3) vers la quatrième vanne (V4) et de commande de la troisième vanne de l'état ouvert (S0) vers l'état fermé (S3) pour pousser le fluide de la troisième vanne (V3) vers la quatrième vanne (V4).

7. Procédé selon la revendication 6, **caractérisé en ce que** la séquence de commande comporte une septième étape de commande de la quatrième vanne (V4), de l'état ouvert (S0) à l'état fermé (S3), pour pousser le fluide vers le canal de sortie microfluidique (101).

8. Système de perfusion microfluidique d'un objet biologique (O), employé pour mettre en oeuvre le procédé de perfusiontel que défini dans l'une des revendications 1 à 7, ledit système comprenant un composant microfluidique (1) intégrant un circuit microfluidique (10), ledit circuit microfluidique ayant
- Un canal principal doté d'un piège hydrodynamique (P_H) recevant ledit objet biologique (O) à perfuser,
- Un canal d'entrée microfluidique (100) connecté sur le canal principal en amont du piège hydrodynamique (P_H),
- Un canal de sortie microfluidique (101) connecté sur le canal principal en aval du piège hydrodynamique (P_H),
- Une première vanne (V1) positionnée sur le canal d'entrée microfluidique (100),
- Un système de pompe formé par une série composée d'au moins une deuxième vanne (V2) et d'une troisième vanne (V3) positionnées sur le canal de sortie microfluidique (101),
- Chaque vanne comportant une cavité (30) et une membrane (33) déformable à l'intérieur de la cavité, ladite membrane (33) étant contrôlable par actionnement pneumatique entre deux états extrêmes, un état ouvert (S0) dans lequel elle laisse passer un fluide et un état fermé (S3) dans lequel elle bloque le passage du fluide, et au moins dans un état intermédiaire, dit deuxième état intermédiaire (S2), situé entre l'état ouvert et l'état fermé,
- Ledit système étant configuré pour exécuter la séquence de commande de la première vanne (V1), de la deuxième vanne (V2) et de la troisième vanne (V3) adaptée pour réaliser le transfert du fluide du canal d'entrée microfluidique (100) vers le canal de sortie microfluidique (101) en passant à travers le piège hydrodynamique (P_H) dans lequel est logé ledit objet biologique pour le perfuser,
- **Caractérisé en ce que**, dans ladite séquence de commande, chaque transfert du fluide de la deuxième vanne (V2) vers la troisième vanne (V3) est réalisé en contrôlant simultanément le déplacement de la membrane de la deuxième vanne (V2) et celui de la membrane de la troisième vanne (V3), la deuxième vanne (V2) étant contrôlée par un premier gradient de pression dans le sens de la fermeture et la troisième vanne (V3) par un deuxième gradient de pression dans le sens de l'ouverture.

9. Système selon la revendication 8, **caractérisé en ce que** le premier gradient de pression et le deuxième gradient de pression sont appliqués de manière symétrique, le premier gradient de pression étant montant et le deuxième gradient de pression étant descendant.

10. Système selon la revendication 8 ou 9, **caractérisé en ce que** le premier gradient de pression et le deuxième gradient de pression sont appliqués chacun suivant un profil rectiligne ou suivant un profil à plusieurs paliers successifs.

11. Système selon l'une des revendications 8 à 10, **caractérisé en ce que**, la première vanne (V1), la deuxième vanne (V2) et la troisième vanne (V3) étant initialement à l'état fermé (S3), ladite séquence de commande comporte :
- Une première étape de commande de la première vanne à l'état ouvert, de la deuxième vanne (V2) et de la troisième vanne (V3) dans un autre état intermédiaire, dit premier état intermédiaire (S1) situé entre l'état ouvert (S0) et le deuxième état intermédiaire (S2), pour pomper le milieu de culture à travers le piège hydrodynamique (P_H),
- Une deuxième étape de commande de la première vanne (V1) à l'état fermé (S3) pour pousser le milieu de culture vers le canal de sortie microfluidique (101), à travers le piège hydrodynamique (P_H),
- Une troisième étape de commande de la deuxième vanne (V2) du premier état intermédiaire (S1) vers le deuxième état intermédiaire (S2) et de la troisième vanne (V3) du premier état intermédiaire (S1) vers l'état ouvert (S0) pour créer ledit transfert de fluide de la deuxième vanne (V2) vers la troisième vanne (V3), cette commande étant réalisée en appliquant sur la deuxième vanne (V2) et la troisième vanne (V3) respectivement le premier gradient de pression et le deuxième gradient de pression, de manière à obtenir pendant un même intervalle de temps une fermeture progressive de la deuxième vanne (V2) et une ouverture progressive de la troisième vanne (V3),
- Une cinquième étape de commande de la deuxième vanne (V2) du deuxième état intermédiaire (S2) vers l'état fermé (S3) pour pousser le fluide vers la troisième vanne (V3) qui est à l'état ouvert (S0).

12. Système selon l'une des revendications 8 à 10, **caractérisé en ce que**, la première vanne (V1), la deuxième vanne (V2) et la troisième vanne (V3) étant initialement à l'état fermé, ladite séquence de commande comporte :
- Une première étape de commande de la première vanne et de la deuxième vanne à l'état ouvert pour pomper le milieu de culture à travers le piège hydrodynamique (P_H),
- Une deuxième étape de commande de la première vanne (V1) à l'état fermé (S3) pour pousser le milieu de culture vers le canal de sortie microfluidique (101), à travers le piège hydrodynamique (P_H),
- Une troisième étape de commande de la troisième vanne (V3) dans le deuxième état intermédiaire (S2),
- Une quatrième étape de commande de la deuxième vanne (V2) de l'état ouvert (S0) vers le deuxième état intermédiaire (S2) et de la troisième vanne (V3) du deuxième état intermédiaire (S2) vers l'état ouvert (S0) pour créer ledit transfert de fluide de la deuxième vanne (V2) vers la troisième vanne (V3), cette commande étant réalisée en appliquant sur la deuxième vanne et la troisième vanne respectivement le premier gradient de pression et le deuxième gradient de pression, de manière à obtenir pendant un même intervalle de temps une fermeture progressive de la deuxième vanne (V2) et une ouverture progressive de la troisième vanne (V3),
- Une cinquième étape de commande de la deuxième vanne (V2) du deuxième état intermédiaire (S2) vers l'état fermé (S3) pour pousser le fluide vers la troisième vanne (V3) qui est à l'état ouvert.

13. Système selon l'une des revendications 8 à 12, **caractérisé en ce que** le système de pompe comporte une quatrième vanne (V4) située en aval de la troisième vanne (V3) et **en ce que** la quatrième vanne (V4) est maintenue à l'état fermé (S3) pendant la première étape de commande, la deuxième étape de commande, la troisième étape de commande, la quatrième étape de commande et la cinquième étape de commande et **en ce que** la séquence de commande comporte :
- Une sixième étape de commande de la quatrième vanne (V4) de l'état fermé (S3) à l'état ouvert (S0) pour autoriser un transfert du fluide de la troisième vanne (V3) vers la quatrième vanne (V4) et de commande de la troisième vanne de l'état ouvert (S0) vers l'état fermé (S3) pour pousser le fluide de la troisième vanne (V3) vers la quatrième vanne (V4).

14. Système selon la revendication 13, **caractérisé en ce que** la séquence de commande comporte une septième étape de commande de la quatrième vanne (V4), de l'état ouvert (S0) à l'état fermé (S3), pour pousser le fluide vers le canal de sortie microfluidique (101).
